# EUROPEAN PATENT SPECIFICATION

(11) **EP 3 885 277 B1**
(45) Date of publication and mention of the grant of the patent: **01.03.2023**
(21) Application number: 21164275.6
(22) Date of filing: 23.03.2021
(51) Int. Cl.: B65B 31/02, B08B 9/032, B65B 55/00, B67C 3/02

(54) **SYSTEM FOR CONVEYING STERILIZING FLUID**
SYSTEM ZUR FÖRDERUNG VON STERILISIERFLÜSSIGKEIT
SYSTÈME DE TRANSPORT DE FLUIDE DE STÉRILISATION

(30) Priority: 26.03.2020 EP 20165919
(43) Date of publication of application: 29.09.2021
(73) Proprietor: Tetra Laval Holdings & Finance S.A., 1009 Pully (CH)
(72) Inventor: DONATI, Andrea, 41051 Castelnuovo Rangone (IT)
(74) Representative: Tetra Pak - Patent Attorneys SE

(56) References cited:
- WO-A2-2014/153520
- US-A- 2 059 455
- US-A- 2 784 748

## Description

### Technical Field

The invention relates to a system and method for conveying a sterilizing fluid to a filling machine.

### Technical Background

A processing system for producing packages filled with liquid food product typically includes a filling machine for forming the packages and filling the packages with the liquid food product. The filling machine may use a sterilizing fluid, such as hydrogen peroxide, for sterilizing both the packaging material and parts of the machine. The sterilizing fluid is circulated around the filling machine through a fluid circuit including a series of tanks, pipes, and process lines for spraying various parts of the filling machine and/or the packaging material. A conventional system for conveying the sterilizing fluid to the filling machine includes using a refilling tank containing the sterilizing fluid and a pump to convey the sterilizing fluid through the fluid circuit to a spray nozzle for the filling machine. Even though the conventional system is both functional and safe, it is desired to provide a more simple system that still assures that sterilizing fluid does not reach parts of the conveying system, or even the filling machine, that are not supposed to come into contact with sterilizing fluid.

WO2014/153520 discloses a filling system for filling a container with a beverage.

### Summary

It is an object of the invention to at least partly overcome one or more limitations of the prior art. In particular, it is an object to provide a system and method that efficiently and safely conveys a sterilizing fluid to a filling machine.

According to an aspect of the invention, a system for conveying a sterilizing fluid to a filling machine includes a refilling tank configured for containing the sterilizing fluid and from which the sterilizing fluid is drawn, a spray tank fluidly connected to the refilling tank for receiving the sterilizing fluid from the refilling tank and conveying the sterilizing fluid to the filling machine, and a fluid line connecting the refilling tank to the spray tank. A vacuum generator is fluidly connected to the spray tank via a vacuum line and configured to generate a vacuum in the spray tank, such that the vacuum causes sterilizing fluid to be drawn from the refilling tank and into the spray tank.

Accordingly, in contrast to conventional systems for conveying the sterilizing fluid to the filling machine, the system described herein advantageously uses a vacuum generator, instead of a pump. The vacuum generator may have a venturi nozzle to generate a vacuum in the spray tank. The system may be switchable between a filling mode and a spraying mode. When the system is in the filling mode, the vacuum generator is in fluid communication with the spray tank to enable drawing of the sterilizing fluid into the spray tank. When the system is switched to a spraying mode, fluid communication between the vacuum generator and the spray tank is closed and the spray tank is pressurized using an air supply. The sterilized fluid may then be forced out of the spray tank to a spray nozzle for the filling machine.

The system may include a plurality of switchable valves that are operable in response to detected levels of the sterilizing fluid and pressure in the spray tank. Using the valves enables the system to accommodate for overpressure and prevent leakage by switching into either the filling mode or the spraying mode in response to the detected fluid levels and pressure. The system may also accommodate for failure in the fluid level sensors or pressure sensors of the spray tank by providing a safety valve that is arranged between the venturi nozzle and the spray tank. In case of failure, the safety valve is configured to stop a feeding of air through the venturi nozzle if the sterilizing fluid enters the vacuum line connected between the vacuum generator and the spray tank.

According to another aspect of the invention, a filling machine is arranged to fill a liquid food product into packages. The filling machine comprises the system for conveying a sterilizing fluid to a filling machine and shares the same advantages.

According to another aspect of the invention, a method for handling a sterilizing fluid to be used for a filling machine includes drawing the sterilizing fluid from a refilling tank, receiving the sterilizing fluid in a spray tank fluidly connected to the refilling tank, and conveying the sterilizing fluid to the filling machine. The drawing step includes generating a vacuum in the spray tank via a vacuum generator fluidly connected to the spray tank.

This method may include the same features as the system for conveying a sterilizing fluid to a filling machine and shares the same advantages.

Still other objectives, features, aspects and advantages of the invention will appear from the following detailed description as well as from the drawings.

### Brief Description of the Drawings

Features of the invention will now be described, by way of example, with reference to the accompanying schematic drawings.
Fig. 1 is a schematic drawing of a system for conveying a sterilizing fluid to a filling machine.
Fig. 2 is a schematic drawing of a vacuum generator of the system of Fig. 1.
Fig. 3 is a schematic drawing of a spray tank of the system of Fig. 1.
Fig. 4 is a schematic drawing of a drain for the system of Fig. 1.
Fig. 5 is a schematic drawing of a filling machine including the system of Fig. 1.
Fig. 6 is a flow chart of a method for conveying a sterilizing fluid to a filling machine using a system for conveying a sterilizing fluid, such as the system of Fig. 1.

### Detailed Description

Embodiments of the invention will now be described more fully hereinafter with reference to the accompanying drawings, in which some, but not all, embodiments of the invention are shown. The invention may be embodied in many different forms and should not be construed as limited to the embodiments set forth herein.

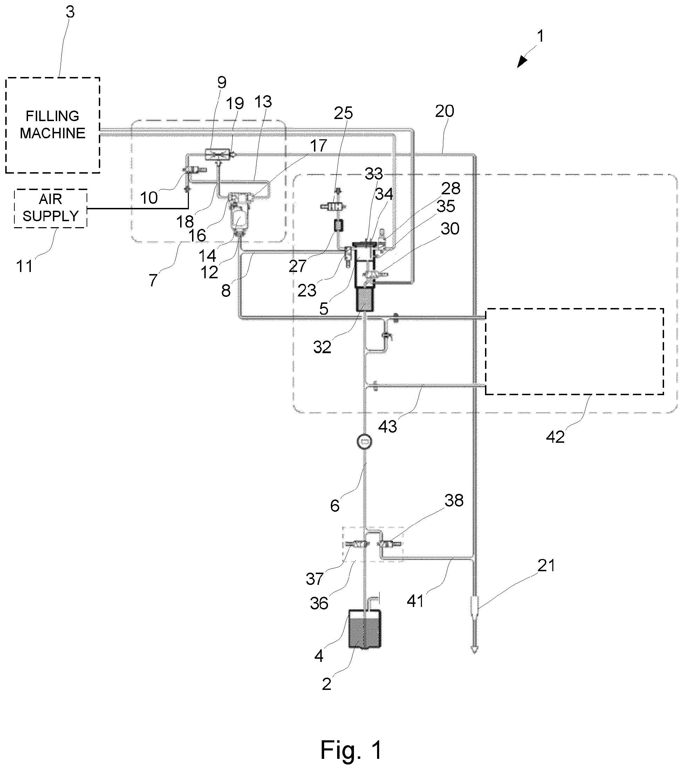

Referring first to Figs. 1-4, a system 1 for conveying a sterilizing fluid 2 to a filling machine 3 is shown. The filling machine 3 is be configured for forming packages and filling the packages with a liquid food product. The sterilizing fluid 2 may be hydrogen peroxide, or any other suitable sterilizing fluid. The system 1 includes a refilling tank 4 for containing the sterilizing fluid 2 and a spray tank 5 fluidly connected to the refilling tank via a fluid line 6. The system 1 includes a fluid circuit formed of a plurality of fluid lines for transporting fluid through the system 1. The fluid may be gas or the sterilizing fluid. The fluid circuit may be formed using any suitable pipes, hoses, tubes, or similar fluid transfer devices. The fluid lines in the system 1 may be connected using any suitable fluid couplers, fittings, or connectors.

The sterilizing fluid 2 is drawn from the refilling tank 4 through the fluid line 6 to the spray tank 5. The system 1 may be oriented vertically such that the refilling tank 4 is arranged at a bottom of the system 1 and the spray tank 5 is arranged at a location above the refilling tank 4. The sterilizing fluid 2 may thus be drawn upwardly. The spray tank 5 is configured for conveying the sterilizing fluid 2 to the filling machine 3. The sterilizing fluid 2 may be conveyed to a spray nozzle of the filling machine 3 that is arranged to spray various parts of the filling machine 3. Nozzles of the filling machine 3 may also be arranged to spray packaging material that is handled by the filling machine 3. The sterilizing fluid 2 is drawn into the spray tank 5 using a vacuum generator 7 that is fluidly connected to the spray tank 5 via a vacuum line 8. The vacuum generator 7 is configured to generate a vacuum in the spray tank 5, such that the vacuum causes the sterilizing fluid 2 to be drawn from the refilling tank 4 as described in further detail below. Herein, "vacuum" refers to a pressure that is below the atmospheric pressure in which the system 1 typically operates, capable of drawing liquid from one tank to another.

The system 1 is operable in a filling mode and a spraying mode that is separate from the filling mode. When the system 1 is in the filling mode, the vacuum generator 7 is in fluid communication with the spray tank 5 for generating the vacuum and drawing the sterilizing fluid 2 into the spray tank 5 to fill the spray tank 5. Fluid communication between the filling machine 3 and the spray tank 5 is closed during the filling mode. When the system 1 is switched to the spraying mode, the fluid communication between the vacuum generator 7 and the spray tank 5 is closed and the spray tank 5 is pressurized. The pressure in the spray tank 5 forces the sterilizing fluid 2 that was drawn into the spray tank 5 during the filling mode to be discharged from the spray tank 5 and conveyed to the spray nozzle for the filling machine 3. Switching the system 1 between the filling mode and the spraying mode may be performed manually, such as by a system operator, or the system 1 may be automated based on the need of sterilization, detected fluid levels and pressure in the system 1.

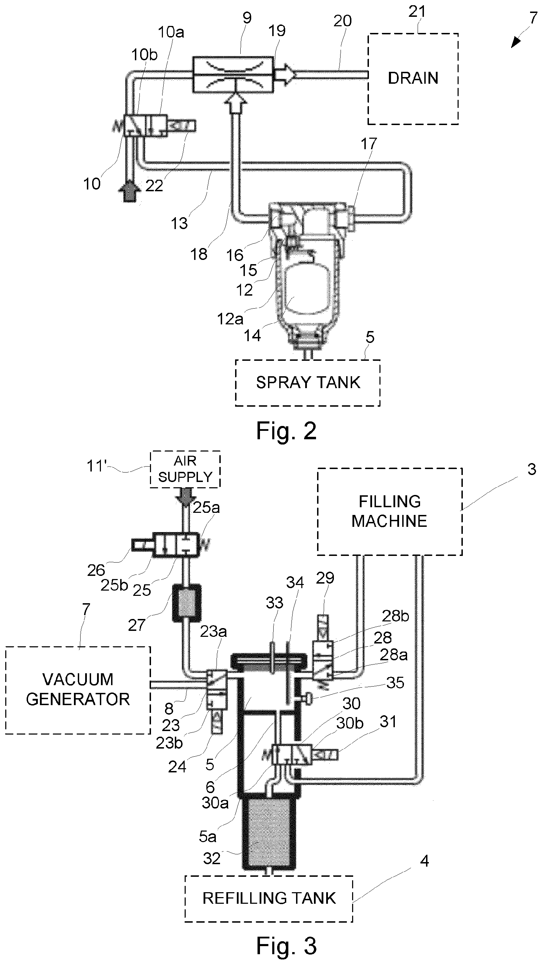

As shown in Fig. 1, and in detail in Fig. 2, the vacuum generator 7 includes a venturi nozzle 9 that is configured to generate a suction or vacuum effect when air is fed through the venturi nozzle 9. An air supply valve 10 is fluidly connected to an air supply 11 and the venturi nozzle 9, as shown in Fig. 1. The air supply valve 10 may be a three-way valve. The air supply 11 is arranged to supply air to the venturi nozzle 9 through the air supply valve 10 when the air supply valve 10 is open to enable fluid communication between the air supply 11 and the venturi nozzle 9. The air supply valve 10 is also connected to a safety valve 12 of the vacuum generator 7 via a vacuum relief line 13 for relieving the vacuum by letting air enter the safety valve 12.

The safety valve 12 is provided to prevent fluid from reaching the venturi nozzle 9 from the spray tank 5 e.g. in the event that fluid level sensors or pressure sensors in the spray tank 5 undergo a failure and the spray tank becomes over-filled, such that the vacuum causes sterilizing fluid to be drawn into the vacuum line 8. The safety valve 12 includes a chamber 12a that is arranged along the vacuum line 8, and a floater 14 located inside the chamber 12a. The floater 14 is configured to rise and close the vacuum line 8 if the sterilizing fluid 2 flows through the vacuum line 8 and enters the chamber 12a. A lever 15 is arranged in the chamber 12a and is movable by the floater 14 for closing a vacuum side port 16 of the safety valve 12 in case sterilizing fluid enters the chamber and causes the floater to rise. When the vacuum side port 16 is closed no vacuum is supplied to the vacuum line 8. This prevents sterilizing fluid from reaching the venturi nozzle 9, which has its vacuum port connected to the vacuum side port 16 of the safety valve 12. A vacuum relief side port 17 of the safety valve 12 is fluidly connected to the air supply valve 10 via the vacuum relief line 13. The air supply valve 10 can be switched to let in air that reaches the vacuum side port 16. In this way resetting of the safety valve 12 is enabled and any sterilizing fluid can be purged from the safety valve 12 and the vacuum line 8, allowing the fluid to flow back towards the spray tank 5.

When the system 1 is in the filling mode, the vacuum side port 16 of the safety valve 12 is open and fluidly connected to the venturi nozzle 9 via a venturi line 18. When air is fed to the venturi nozzle 9 from the air supply 11, the vacuum is generated in the venturi line 18 and further down in the safety valve 12, the vacuum line 8 and the spray tank 5. An air outlet 19 of the venturi nozzle 9 is connected to an air outlet line 20 which is connected between the venturi nozzle 9 and a drain 21 for the system 1. Air that flows through the venturi nozzle 9 to generate the vacuum leaves the system 1 via the drain 21.

When the system 1 is in the filling mode, the air supply valve 10 is in a first position 10a, in which fluid communication between the air supply 11 and the venturi nozzle 9 is open. The air supply valve 10 may be normally biased in a second position 10b and energized by a solenoid 22 to move to the first position 10a. When in the first position 10a, air from the air supply 11 is able to flow to the venturi nozzle 9. The vacuum relief line 13 is closed by the air supply valve 10 when in the first position 10a. The vacuum effect is generated in the venturi nozzle 9 and is effectively generated upstream through the venturi line 18, the open vacuum side port 16 of the safety valve 12, the chamber 12a of the safety valve 12, and the vacuum line 8, to the spray tank 5. The vacuum effect thus occurs upwardly through the system 1 toward the venturi nozzle 9. When the air supply valve 10 is in the second position it provides communication between the vacuum relief line 13 and the venturi nozzle, and thereby also to the drain 21. Air may then enter form the drain and eventually flow into the chamber 12a of the safety valve. This enables the resetting of the safety valve 12, allowing any fluid therein to flow back towards the spray tank 5. For this purpose the spray tank is located vertically below the safety valve 12. Alternatively, pressurized air may be fed into the vacuum relief line 13 for pushing liquid back towards the spray tank 5.

As shown in Fig. 1, and in detail in Fig. 3, a pressure control valve 23 is connected between the vacuum line 8 and the spray tank 5 for enabling and preventing fluid communication between the vacuum generator 7 and the spray tank 5. The pressure control valve 23 may be a three-way pilot-operated switching valve. The pressure control valve 23 is a pressure control valve in the sense that it is used for controlling whether vacuum or pressurized air shall be supplied the spray tank 5, i.e. if under-atmospheric pressure or overatmospheric pressure is provided for the spray tank 5. The pressure control valve 23 may be operable by a solenoid 24. When the system 1 is in the filling mode, the pressure control valve 23 is in a first position 23a in which the pressure control valve 23 is open between the vacuum line 8 and the spray tank 5 to enable generation of the vacuum in the spray tank 5. When the system 1 is switched to the spraying mode, the pressure control valve 23 is energized to move from the first position 23a to a second position 23b in which the pressure control valve 23 closes the vacuum line 8 and thus the vacuum generator 7 relative to the spray tank 5. When in the second position 23b, the pressure control valve 23 enables a supply of pressurized air 11' to be in fluid communication with the spray tank 5, such that the spray tank 5 becomes pressurized.

The air supply is conveyed to the spray tank 5 from an air supply 11' through an air supply valve 25. The air supply valve 25 may be a solenoid valve operable by a solenoid 26. The air supply valve 25 is normally biased in a first position 25a where the valve 25 is closed, and movable to a second and open position 25b when the solenoid 26 is energized. During the filling mode of the system 1, the air supply valve 25 is in the closed position 25a, such that the air supply valve 25 is in the closed position 25a while the pressure control valve 23 is in the first position 23a to enable fluid communication between the vacuum generator 7 and the spray tank 5. When the system 1 is switched to the spraying mode, the pressure control valve 23 is energized to move to the second position 23b and the air supply valve 25 is moved to the open position 25b to open the fluid communication between the supply of pressurized air 11' and the spray tank 5. Then the pressure control valve 23 is in the second position 23b, enabling the supply of pressurized air 11' to pressurize the spray tank 5. A filter 27 may be arranged between the air supply valve 25 and the pressure control valve 23 to ensure that only clean air enters the spray tank 5.

An air-conveying valve 28 is connected between the spray tank 5 and the filling machine 3 for conveying pressurized air to the filling machine 3 during the spraying mode of the system 1. The air-conveying valve 28 may be a pilot-operated switching valve. The air-conveying valve 28 is operable by a solenoid 29 and normally biased in a closed position 28a in which fluid communication between the spray tank 5 and the filling machine 3 is closed by the air-conveying valve 28. The air-conveying valve 28 is in the closed position 28a when the system 1 is in the filling mode. When the system 1 is switched to the spraying mode, the air-conveying valve 28 is energized to move to an open position 28b in which fluid communication between the spray tank 5 and the filling machine 3 is open such that pressurized air in the spray tank 5 flows out of the spray tank 5 and toward the spraying nozzle of the filling machine 3. The pressurized air is mixed with sterilizing fluid at the spray nozzle to generate the spray of sterilizing fluid for spraying.

A sterilizing fluid control valve 30 is arranged along the fluid line 6 which extends from the refilling tank 4 to the spray tank 5. The sterilizing fluid control valve 30 is configured for conveying the sterilizing fluid 2 from the spray tank 5 to the filling machine 3. The sterilizing fluid control valve 30 may be a three-way pilot operated switching valve and is supported by a housing 5a that houses the spray tank 5. The sterilizing fluid control valve 30 may be operated by a solenoid 31 and normally biased in a first position 30a. When the system 1 is in the filling mode, the sterilizing fluid control valve 30 is in the first position 30a in which the sterilizing fluid control valve 30 enables fluid communication between the fluid line 6 and the spray tank 5 to be open such that the sterilizing fluid 2 may be drawn into the spray tank 5 from the refilling tank 4. A filter 32 may also be arranged along the fluid line 6 to ensure that only clean sterilizing fluid enters the spray tank 5.

When the system 1 is switched to the spraying mode, the sterilizing fluid control valve 30 is energized to move to a second position 30b in which fluid communication between the refilling tank 4 and the spray tank 5 is closed and fluid communication between the spray tank 5 and the filling machine 3 is opened by the sterilizing fluid control valve 30. When the spray tank 5 is pressurized, the sterilizing fluid 2 that was drawn into the spray tank 5 during the filling mode is pushed out of the spray tank 5 through the sterilizing fluid control valve 30. The sterilizing fluid 2 is conveyed to the spray nozzle for the filling machine 3 for mixing with the pressurized air that is supplied via the air-conveying valve 28. Thus, when the sterilizing fluid control valve 30 is in the second position 30b, the air-conveying valve 28 is in the open position 28b such that the air and sterilizing fluid are simultaneously conveyed to the spray nozzle. The sterilizing fluid level in the spray tank 5 is regulated such that the outlet to the air-conveying valve 28 from the spray tank 5 remains above the fluid level. The outlet to the sterilizing fluid control valve 30 is located at the bottom of the spray tank 5.

Switching the system 1 between the filling mode and the spraying mode may be performed in response to the fluid level in the spray tank 5. Separate fluid level sensors or relays 33, 34 may be mounted in the spray tank 5 for detecting a high level and low level of the sterilizing fluid 2 in the spray tank 5, respectively. A pressure transducer 35 is also attached to the spray tank 5 for monitoring the pressure in the spray tank 5. More than one pressure transducer may be provided and the pressure transducers may be arranged at any suitable location along the spray tank 5. The air supply valve 25 may be controlled in response to the pressure detected by the pressure transducer 35. The air supply valve 25 may be energized to move to the open position 25b if low pressure is detected or to the closed position 25a if high pressure is detected. The pressure transducer 35 may also be used to prevent leakages by detecting varying amounts of pressure that occur due to insufficient tightening of fluid connectors or fittings in the fluid circuit.

The relays 33, 34 include a high level relay 33 for detecting a high level of sterilizing fluid 2 and a low level relay 34 for detecting a low level of sterilizing fluid 2. When a low level of the sterilizing fluid 2 is detected, or a level of the sterilizing fluid 2 that is below a predetermined threshold is detected, the system 1 may be switched to the filling mode such that the vacuum generator 7 is operated and connected for fluid communication with the spray tank 5. The air supply valve 25 is thus moved to the closed position 25a, the air-conveying valve 28 is moved to the first position 28a, the pressure control valve 23 is moved to the first position 23a, and the sterilizing fluid control valve 30 is moved to the first position 30a. The air supply valve 10 of the vacuum generator 7 is also moved to the first position 10a. The valves may be simultaneously energized to move to the respective positions.

When the high level relay 33 detects that the level of the sterilizing fluid 2 in the spray tank 5 reaches a predetermined threshold, the system 1 may be switched to the spraying mode in which fluid communication between the vacuum generator 7 and the spray tank 5 is closed such that the sterilizing fluid 2 is no longer drawn into the spray tank 5. The air supply valve 25 is moved to the open position 25b, the pressure control valve 23 is moved to the second position 23b, the air-conveying valve 28 is moved to the second position 28b, and the sterilizing fluid control valve 30 is moved to the second position 30b. The air supply valve 10 of the vacuum generator 7 is moved into the second position 10b for opening the vacuum relief line 13 such that the vacuum from the venturi nozzle 9 is relieved. The vacuum relief line 13 is connected to the vacuum relief side port 17 of the safety valve 12 which is open such that opening the vacuum relief line 13 enables atmospheric pressure to be generated in the vacuum line 8 when no air is being fed to the venturi nozzle 9.

If the relays 33, 34 or the pressure transducer 30 malfunction such that the system 1 continues in the filling mode even though the spray tank 5 is full, the sterilizing fluid 2 may enter into the vacuum line 8. If the sterilized fluid 2 reaches the chamber 12a of the safety valve 12 arranged along the vacuum line 8, the floater 14 of the safety valve 12 is moved upwardly in the chamber 12a of the safety valve 12a to engage the lever 15 and close the vacuum side port 16 of the safety valve 12. The venturi line 18 to the venturi nozzle 9 is thus also closed. This prevents further filling of sterilizing fluid into the safety valve 12, so that no fluid reaches the venturi nozzle 9. The system 1 may be shut off and restarted after this happens. The floater 14 may be pushed downwardly to force the sterilizing fluid 2 back through the vacuum line 8 and into the spray tank 5 by switching the air supply valve 10 to the second position 10b. The lever 15 may be a manual valve that is manually operated by the upward and downward movement of the floater 14.

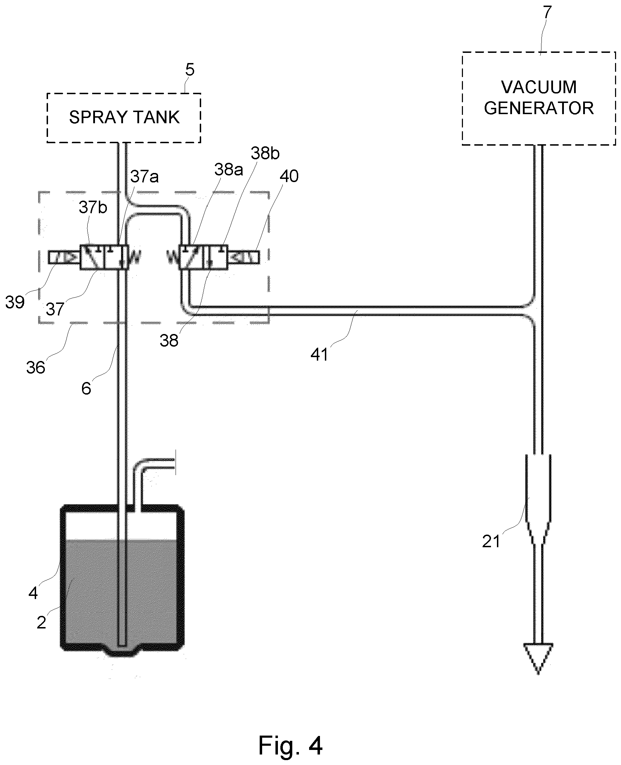

As shown in Fig. 1, and in detail in Fig. 4, the sterilizing fluid 2 and air from the venturi nozzle 9 may be drained from the system 1 via the drain 21 during a draining mode. The sterilizing fluid 2 may be drained to the same outlet line 20 as the discharged air. A separate drain line that is fluidly connected to the drain 21 may also be provided. A manifold structure 36 is arranged along the fluid line 6 and includes a loading valve 37 and a drain valve 38. The loading valve 37 may be a three-way pilot-operated valve having a solenoid 39. The loading valve 37 may be normally biased in a first position 37a in which the loading valve 37 is open and enables fluid communication between the refilling tank 4 and the spray tank 5. During the filling mode of the system 1, the loading valve 37 is open and the drain valve 38 is in a closed position 38a.

During the draining mode, the loading valve 37 is energized to move into a second position 37b in which the loading valve 37 is closed relative to the refilling tank 4 and sterilizing fluid 2 is no longer drawn from the refilling tank 4. The loading valve 37 is also in the second position 37b during the spraying mode of the system 1. In the drain mode, when the loading valve 37 is closed, the drain valve 38 is energized to move to an open position 38b. The drain valve 38 may be a three-way pilot-operated valve having a solenoid 40 and is normally biased in the closed position 38a. When the loading valve 37 is in the second position 37b and the drain valve 38 is in the open position 38b, the sterilizing fluid 2 flows downwardly through the fluid line 6 and through the drain valve 38 toward the drain 21. When this happens the air supply valve 25 and the pressure control valve 23 are in their positions to pressurize the spray tank 5 with air from air supply 11'. The air-conveying valve 28 and the fluid control valve 30 are then closed, such that the pressurized air can assist in draining the spray tank 5 towards the fluid line 6 and further to the drain valve 38 and the drain 21. A fluid drain line 41 is connected between the fluid line 6 and the drain 21 and the drain valve 38 is arranged along the fluid drain line 41.

The sterilizing fluid 2 may be emptied from the refilling tank 4 by repeatedly switching between the filling mode and the draining mode. The loading valve 37 and the drain valve 38 may be repeatedly opened and closed until all of the sterilizing fluid 2 is discharged to the drain 21. The system 1 is initially in the filling mode to draw the sterilizing fluid 2 from the refilling tank 4. When the system 1 is in the filling mode, the air supply valve 25 is in the closed position 25a, the air-conveying valve 28 is in the first position 28a, the sterilizing fluid control valve 30 is in the first position 30a, and the pressure control valve 23 is in the first position 23a. When the spray tank 5 is filled to a predetermined level as detected by the high level relay 34 and the pressure transducer 35, the system 1 is switched into the draining mode. The filling and draining sequence may repeat until all of the sterilizing fluid 2 is removed from the system 1.

The system 1 may also be configured for an operational mode in which sterilizing fluid is supplied to another unit 42 where sterilization is needed. When the system 1 is switched into the operational mode for supplying fluid to the other unit 42, the pressure control valve 23 may be moved to the second position 23b in which the vacuum generator 7 is disconnected from the spray tank 5. The vacuum generator 7 and vacuum line 8 may then be fluidly connected to the other unit 42 for generating a vacuum in the unit 42. The sterilizing fluid control valve 30 is moved to the second position 30b such that the sterilizing fluid 2 is drawn into the unit 42, rather than to the spray tank 5. The sterilizing fluid 2 may be drawn into the unit 42 via an injection fluid line 43 that is fluidly connected to the fluid line 6.

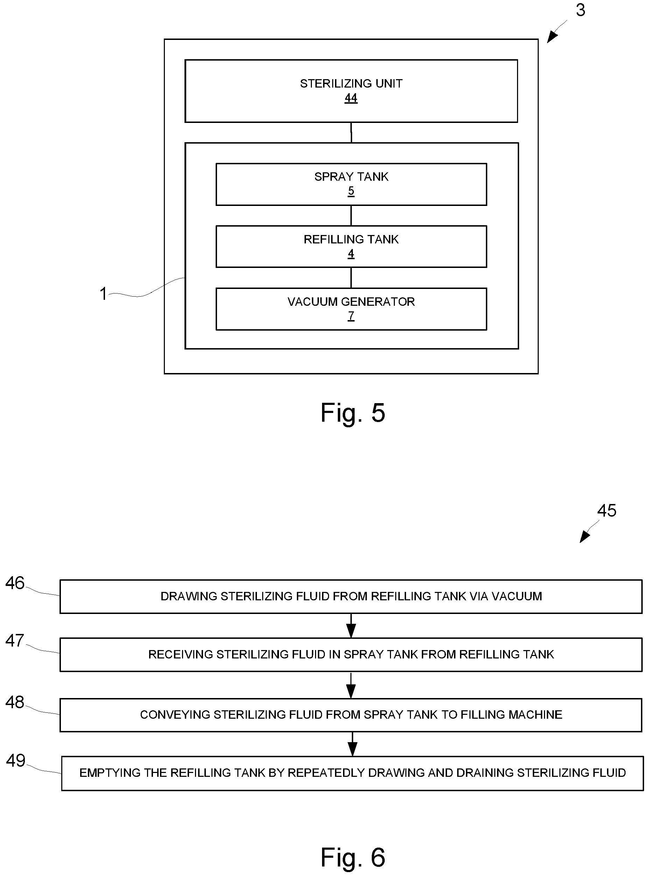

Referring now to Figs. 5 and 6, the filling machine 3 may include the system 1 described herein and a sterilizing unit 44 arranged to receive the sterilizing fluid 2 from the system 1 for sterilizing parts of the filling machine 3. A method 45 for handling a sterilizing fluid 2 to be used for a filling machine 3 is shown in a flowchart in Fig. 6. The method 45 may be performed using the filling machine 3 of Fig. 5. A step 46 of the method 45 includes drawing the sterilizing fluid 2 from a refilling tank 4. Step 46 includes generating a vacuum in the spray tank 5 via a vacuum generator 7 fluidly connected to the spray tank 5. A step 47 includes receiving the sterilizing fluid 2 in a spray tank 5 that is fluidly connected to the refilling tank 4. A step 48 includes conveying the sterilizing fluid 2 to the filling machine 3. A step 49 of the method 44 includes emptying the refilling tank 4 by repeatedly drawing the sterilizing fluid 2 into the spray tank 5 and draining the sterilizing fluid 2 out of the spray tank 5 until the refilling tank 4 is empty.

The system and method for conveying a sterilizing fluid to a filling machine is advantageous in that the system efficiently draws a sufficient amount of sterilizing fluid from the refilling tank to supply the fluid downstream and spray the filling machine. Another advantage of the system is that pressure sensors and separate fluid level sensors are used to detect real-time characteristics of the spray tank such that the system may accommodate for overpressure and leakage. The vacuum generator advantageously includes a safety valve for the venturi nozzle that prevents a feed of air to the venturi nozzle during a failure of the fluid level sensors or the pressure sensors for the spray tank. Still another advantage of the system is that the refilling tank may be easily emptied by a repeated process of drawing the sterilizing fluid into the spray tank and discharging the sterilizing fluid to the drain.

From the description above follows that, although various embodiments of the invention have been described and shown, the invention is not restricted thereto, but may also be embodied in other ways within the scope of the subject-matter defined in the following claims.

## Claims

1. A filling machine (3) arranged to fill liquid food product into packages, wherein the filling machine (3) comprises a system (1) for conveying a sterilizing fluid (2) to the filling machine (3), **characterized in that** the system (1) comprises:
a refilling tank (4) configured for containing the sterilizing fluid (2) and from which the sterilizing fluid (2) is drawn,
a spray tank (5) fluidly connected to the refilling tank (4) for receiving the sterilizing fluid (2) from the refilling tank (4) and conveying the sterilizing fluid (2) to the filling machine (3), wherein the filling machine (3) comprises a spray nozzle configured for spraying various parts of the filling machine (3), wherein the spray tank (5) is configured for conveying the sterilizing fluid to said spray nozzle,
a fluid line (6) connecting the refilling tank (4) to the spray tank (5), and
a vacuum generator (7) fluidly connected to the spray tank (5) via a vacuum line (8) and configured to generate a vacuum in the spray tank (5), such that the vacuum causes the sterilizing fluid (2) to be drawn from the refilling tank (4) and into the spray tank (5).

2. The filling machine (3) according to claim 1, wherein the vacuum generator (7) comprises a venturi nozzle (9) configured to generate the vacuum when air is fed through the venturi nozzle (9).

3. The filling machine (3) according to claim 2, wherein the vacuum generator (7) comprises a safety valve (12) configured to stop a feeding of air through the venturi nozzle (9) if the sterilizing fluid (2) enters the vacuum line (8).

4. The filling machine (3) according to claim 3, wherein the safety valve (12) comprises a chamber (12a) that is arranged along the vacuum line (8), and a floater (14) located inside the chamber (12a) and configured to rise and close the vacuum line (8) if the sterilizing fluid (2) enters the chamber (12a).

5. The filling machine (3) according to claim 3 or 4, wherein the vacuum generator (7) comprises:
an air supply valve (10) connected to the venturi nozzle (9) for feeding air to the venturi nozzle (9), and
a vacuum relief line (13) connected between the air supply valve (10) and the safety valve (12) for relieving the vacuum in the vacuum generator (7) and enable resetting of the safety valve (12) in case it has released.

6. The filling machine (3) according to claim 5, wherein the air supply valve (10) is configured to move between
- a first position (10a) that enables the air to be fed to the venturi nozzle (9) while closing the vacuum relief line (13), and
- a second position (10b) that prevents air from being fed to the venturi nozzle (9) while opening the vacuum relief line (13), thereby enabling atmospheric pressure in the vacuum relief line (13).

7. The filling machine (3) according to any preceding claim, comprising:
a drain (21) configured for discharging sterilizing fluid from the system (1),
a loading valve (37) connected between the refilling tank (4) and the spray tank (5) for enabling the sterilizing fluid (2) to be drawn into the spray tank (5) when the loading valve (37) is open, and
a fluid drain line (41) connected between the refilling tank (4) and the drain (21) for discharging the sterilizing fluid (2) from the spray tank (5) when the loading valve (37) is closed.

8. The filling machine (3) according to claim 7, comprising a drain valve (38) arranged along the fluid drain line (41) for emptying the refilling tank (4) when the drain valve (38) is open and the loading valve (37) is closed and, wherein the venturi nozzle (9) has an air outlet (19) connected to the drain (21) via an air outlet line (20) for discharging air from the venturi nozzle (9).

9. The filling machine (3) according to any preceding claim, wherein said filling machine (3) is configured for forming packages and filling the packages with the liquid food product.

10. The filling machine (3) according to any preceding claim, wherein the spray tank (5) includes a pressure transducer (35) configured to monitor a pressure inside the spray tank (5).

11. The filling machine (3) according to any preceding claim, wherein the spray tank (5) includes a high level relay (33) and a low level relay (34) that are each configured to detect a level of the sterilizing fluid (2) in the spray tank (5).

12. The filling machine (3) according to any preceding claim, comprising:
a pressure control valve (23) for the spray tank (5), configured to move between
- a first position (23a) that provides a connection between the spray tank (5) and the vacuum generator (7), for allowing the vacuum in the spray tank (5) to be generated, and
- a second position (23b) that provides a connection between the spray tank (5) and an air supply valve (25), for allowing pressurized air to enter the spray tank (5), to be used for conveying a sterilizing fluid (2), and
a sterilizing fluid control valve (30) that is configured to move between
- a first position (30a) that provides a connection between the spray tank (5) and the refilling tank (4), for allowing the sterilizing fluid (2) to be drawn from the refilling tank (4) and into the spray tank (5), and
- a second position (30b) that provides a connection between the spray tank (5) and the filling machine (3), for allowing the sterilizing fluid (2) to be conveyed to the filling machine (3).

13. The filling machine (3) according to any preceding claim, wherein the system (1) is operable in a filling mode and in a spraying mode that is separate from the filling mode, wherein:
in the filling mode, the vacuum generator (7) is in fluid communication with the spray tank (5) for generating the vacuum and drawing the sterilizing fluid (2) into the spray tank (5) to fill the spray tank (5), and fluid communication between the filling machine (3) and the spray tank (5) is closed;
in the spraying mode, the fluid communication between the vacuum generator (7) and the spray tank (5) is closed and the spray tank (5) is pressurized, so that pressure in the spray tank (5) forces the sterilizing fluid (2) that was drawn into the spray tank (5) during the filling mode to be discharged from the spray tank (5) and conveyed to the spray nozzle for the filling machine (3).

14. A method (45) for handling a sterilizing fluid (2) to be used for a filling machine (3) that is arranged to fill a liquid food product into packages, the method (45) comprising:
drawing (46) the sterilizing fluid (2) from a refilling tank (4),
receiving (47) the sterilizing fluid (2) in a spray tank (5) fluidly connected to the refilling tank (4), and
conveying (48) the sterilizing fluid (2) to the filling machine (3),
wherein the sterilizing fluid (2) is conveyed to a spray nozzle of the filling machine (3) that is arranged to spray various parts of the filling machine (3),
wherein the drawing (46) comprises generating a vacuum in the spray tank (5) via a vacuum generator (7) fluidly connected to the spray tank (5).

15. The method (45) according to claim 14, wherein the sterilizing fluid (2) is hydrogen peroxide.

## Patentansprüche

1. Füllmaschine (3), die dazu angeordnet ist, flüssiges Nahrungsmittelprodukt in Verpackungen zu füllen, wobei die Füllmaschine (3) ein System (1) zum Leiten eines Sterilisationsfluids (2) zu der Füllmaschine (3) umfasst, **dadurch gekennzeichnet, dass** das System (1) Folgendes umfasst:
einen Nachfülltank (4), der dazu ausgelegt ist, das Sterilisationsfluid (2) zu enthalten, und aus dem das Sterilisationsfluid (2) gesaugt wird,
einen Sprühtank (5), der mit dem Nachfülltank (4) strömungstechnisch verbunden ist, um das Sterilisationsfluid (2) aus dem Nachfülltank (4) aufzunehmen und das Sterilisationsfluid (2) zu der Füllmaschine (3) zu leiten, wobei die Füllmaschine (3) eine Sprühdüse umfasst, die dazu ausgelegt ist, verschiedene Teile der Füllmaschine (3) zu besprühen, wobei der Sprühtank (5) dazu ausgelegt ist, das Sterilisationsfluid zu der Sprühdüse zu leiten,
eine Fluidleitung (6), die den Nachfülltank (4) mit dem Sprühtank (5) verbindet, und
einen Unterdruckerzeuger (7), der mit dem Sprühtank (5) über eine Unterdruckleitung (8) strömungstechnisch verbunden und dazu ausgelegt ist, einen Unterdruck in dem Sprühtank (5) zu erzeugen, so dass der Unterdruck bewirkt, dass das Sterilisationsfluid (2) aus dem Nachfülltank (4) und in den Sprühtank (5) gesaugt wird.

2. Füllmaschine (3) nach Anspruch 1, wobei der Unterdruckerzeuger (7) eine Venturidüse (9) umfasst, die dazu ausgelegt ist, den Unterdruck zu erzeugen, wenn Luft durch die Venturidüse (9) gespeist wird.

3. Füllmaschine (3) nach Anspruch 2, wobei der Unterdruckerzeuger (7) ein Sicherheitsventil (12) umfasst, das dazu ausgelegt ist, ein Speisen von Luft durch die Venturidüse (9) zu stoppen, wenn das Sterilisationsfluid (2) in die Unterdruckleitung (8) eintritt.

4. Füllmaschine (3) nach Anspruch 3, wobei das Sicherheitsventil (12) eine Kammer (12a), die entlang der Unterdruckleitung (8) angeordnet ist, und einen Schwimmer (14), der sich in der Kammer (12a) befindet und dazu ausgelegt ist, zu steigen und die Unterdruckleitung (8) zu schließen, wenn das Sterilisationsfluid (2) in die Kammer (12a) eintritt, umfasst.

5. Füllmaschine (3) nach Anspruch 3 oder 4, wobei der Unterdruckerzeuger (7) Folgendes umfasst:
ein Luftzufuhrventil (10), das mit der Venturidüse (9) zum Speisen von Luft zu der Venturidüse (9) verbunden ist, und
eine Unterdruckabbauleitung (13), die zwischen dem Luftzufuhrventil (10) und dem Sicherheitsventil (12) verbunden ist, um den Unterdruck in dem Unterdruckerzeuger (7) abzubauen und ein Rücksetzen des Sicherheitsventils (12) zu ermöglichen, falls es ausgelöst hat.

6. Füllmaschine (3) nach Anspruch 5, wobei das Luftzufuhrventil (10) dazu ausgelegt ist, sich zwischen Folgendem zu bewegen
- einer ersten Position (10a), die ermöglicht, dass die Luft zu der Venturidüse (9) gespeist wird, während die Unterdruckabbauleitung (13) geschlossen wird, und
- einer zweiten Position (10b), die verhindert, dass Luft zu der Venturidüse (9) gespeist wird, während die Unterdruckabbauleitung (13) geöffnet wird, wodurch atmosphärischer Druck in der Unterdruckabbauleitung (13) ermöglicht wird.

7. Füllmaschine (3) nach einem der vorhergehenden Ansprüche, umfassend:
einen Ablauf (21), der dazu ausgelegt ist, Sterilisationsfluid aus dem System (1) abzulassen,
ein Ladeventil (37), das zwischen dem Nachfülltank (4) und dem Sprühtank (5) verbunden ist, um zu ermöglichen, dass das Sterilisationsfluid (2) in den Sprühtank (5) gesaugt wird, wenn das Ladeventil (37) geöffnet ist, und
eine Fluidablaufleitung (41), die zwischen dem Nachfülltank (4) und dem Ablauf (21) verbunden ist, um das Sterilisationsfluid (2) aus dem Sprühtank (5) abzulassen, wenn das Ladeventil (37) geschlossen ist.

8. Füllmaschine (3) nach Anspruch 7, umfassend ein Ablaufventil (38), das entlang der Fluidablaufleitung (41) angeordnet ist, um den Nachfülltank (4) zu leeren, wenn das Ablaufventil (38) geöffnet ist und das Ladeventil (37) geschlossen ist, und wobei die Venturidüse (9) einen Luftauslass (19) aufweist, der mit dem Ablauf (21) über eine Luftauslassleitung (20) verbunden ist, um Luft aus der Venturidüse (9) abzulassen.

9. Füllmaschine (3) nach einem der vorhergehenden Ansprüche, wobei die Füllmaschine (3) dazu ausgelegt ist, Verpackungen auszubilden und die Verpackungen mit dem flüssigen Nahrungsmittelprodukt zu füllen.

10. Füllmaschine (3) nach einem der vorhergehenden Ansprüche, wobei der Sprühtank (5) einen Druckwandler (35) umfasst, der dazu ausgelegt ist, einen Druck in dem Sprühtank (5) zu überwachen.

11. Füllmaschine (3) nach einem der vorhergehenden Ansprüche, wobei der Sprühtank (5) ein Hochpegelrelais (33) und ein Niederpegelrelais (34) umfasst, die jeweils dazu ausgelegt sind, einen Pegel des Sterilisationsfluids (2) in dem Sprühtank (5) zu detektieren.

12. Füllmaschine (3) nach einem der vorhergehenden Ansprüche, umfassend:
ein Drucksteuerventil (23) für den Sprühtank (5), das dazu ausgelegt ist, sich zwischen Folgendem zu bewegen
- einer ersten Position (23a), die eine Verbindung zwischen dem Sprühtank (5) und dem Unterdruckerzeuger (7) bereitstellt, um zu ermöglichen, dass der Unterdruck in dem Sprühtank (5) erzeugt wird, und
- einer zweiten Position (23b), die eine Verbindung zwischen dem Sprühtank (5) und einem Luftzufuhrventil (25) bereitstellt, um zu ermöglichen, dass Druckluft in den Sprühtank (5) eintritt, um zum Leiten eines Sterilisationsfluids (2) verwendet zu werden, und
ein Sterilisationsfluidsteuerventil (30), das dazu ausgelegt ist, sich zwischen Folgendem zu bewegen
- einer ersten Position (30a), die eine Verbindung zwischen dem Sprühtank (5) und dem Nachfülltank (4) bereitstellt, um zu ermöglichen, dass das Sterilisationsfluid (2) aus dem Nachfülltank (4) und in den Sprühtank (5) gesaugt wird, und
- einer zweiten Position (30b), die eine Verbindung zwischen dem Sprühtank (5) und der Füllmaschine (3) bereitstellt, um zu ermöglichen, dass das Sterilisationsfluid (2) zu der Füllmaschine (3) geleitet wird.

13. Füllmaschine (3) nach einem der vorhergehenden Ansprüche, wobei das System (1) in einem Füllmodus und in einem Sprühmodus, der von dem Füllmodus separat ist, betreibbar ist, wobei:
in einem Füllmodus der Unterdruckerzeuger (7) in strömungstechnischer Verbindung mit dem Sprühtank (5) ist, um den Unterdruck zu erzeugen und das Sterilisationsfluid (2) in den Sprühtank (5) zu saugen, um den Sprühtank (5) zu füllen, und eine strömungstechnische Verbindung zwischen der Füllmaschine (3) und dem Sprühtank (5) geschlossen ist;
in dem Sprühmodus die strömungstechnische Verbindung zwischen dem Unterdruckerzeuger (7) und dem Sprühtank (5) geschlossen ist und der Sprühtank (5) druckbeaufschlagt ist, so dass Druck in dem Sprühtank (5) das Sterilisationsfluid (2), das während des Füllmodus in den Sprühtank (5) gesaugt wurde, zwingt, aus dem Sprühtank (5) ausgelassen und zu der Sprühdüse für die Füllmaschine (3) geleitet zu werden.

14. Verfahren (45) zum Handhaben eines Sterilisationsfluids (2), das für eine Füllmaschine (3) verwendet werden soll, die dazu angeordnet ist, flüssiges Nahrungsmittelprodukt in Verpackungen zu füllen, wobei das Verfahren (45) Folgendes umfasst:
Saugen (46) des Sterilisationsfluids (2) aus einem Nachfülltank (4),
Aufnehmen (47) des Sterilisationsfluids (2) in einem Sprühtank (5), der strömungstechnisch mit dem Nachfülltank (4) verbunden ist, und
Leiten (48) des Sterilisationsfluids (2) zu der Füllmaschine (3),
wobei das Sterilisationsfluid (2) zu einer Sprühdüse der Füllmaschine (3) geleitet wird, die dazu angeordnet ist, verschiedene Teil der Füllmaschine (3) zu besprühen,
wobei das Saugen (46) Erzeugen eines Unterdrucks in dem Sprühtank (5) über einen Unterdruckerzeuger (7), der strömungstechnisch mit dem Sprühtank (5) verbunden ist, umfasst.

15. Verfahren (45) nach Anspruch 14, wobei das Sterilisationsfluid (2) Wasserstoffperoxid ist.

## Revendications

1. Machine à emplir (3) agencée pour emplir des emballages d'un produit alimentaire liquide, la machine à emplir (3) comprenant un système (1) pour transporter un fluide de stérilisation (2) vers la machine à emplir (3), **caractérisée en ce que** le système (1) comprend :
un réservoir de remplissage (4) configuré pour contenir le fluide de stérilisation (2) et duquel le fluide de stérilisation (2) est aspiré,
un réservoir de pulvérisation (5) raccordé de façon fluidique au réservoir de remplissage (4) pour recevoir le fluide de stérilisation (2) du réservoir de remplissage (4) et transporter le fluide de stérilisation (2) vers la machine à emplir (3), la machine à emplir (3) comprenant une buse de pulvérisation configurée pour pulvériser diverses parties de la machine à emplir (3), le réservoir de pulvérisation (5) étant configuré pour transporter le fluide de stérilisation vers ladite buse de pulvérisation,
une conduite de fluide (6) raccordant le réservoir de remplissage (4) au réservoir de pulvérisation (5), et
un générateur de vide (7) raccordé de façon fluidique au réservoir de pulvérisation (5) par l'intermédiaire d'une conduite de vide (8) et configuré pour générer un vide dans le réservoir de pulvérisation (5), de telle sorte que le vide amène le fluide de stérilisation (2) à être aspiré du réservoir de remplissage (4) et jusque dans le réservoir de pulvérisation (5).

2. Machine à emplir (3) selon la revendication 1, dans laquelle le générateur de vide (7) comprend une buse venturi (9) configurée pour générer le vide lorsque de l'air est apporté à travers la buse venturi (9).

3. Machine à emplir (3) selon la revendication 2, dans laquelle le générateur de vide (7) comprend une valve de sécurité (12) configurée pour arrêter un apport d'air à travers la buse venturi (9) si le fluide de stérilisation (2) entre dans la conduite de vide (8).

4. Machine à emplir (3) selon la revendication 3, dans laquelle la valve de sécurité (12) comprend une chambre (12a) qui est agencée le long de la conduite de vide (8), et un flotteur (14) situé à l'intérieur de la chambre (12a) et configuré pour monter et fermer la conduite de vide (8) si le fluide de stérilisation (2) entre dans la chambre (12a).

5. Machine à emplir (3) selon la revendication 3 ou la revendication 4, dans laquelle le générateur de vide (7) comprend :
une valve d'alimentation en air (10) raccordée à la buse venturi (9) pour apporter de l'air vers la buse venturi (9), et
une conduite de décharge de vide (13) raccordée entre la valve d'alimentation en air (10) et la valve de sécurité (12) pour décharger le vide dans le générateur de vide (7) et assurer la réinitialisation de la valve de sécurité (12) dans le cas où elle s'est déclenchée.

6. Machine à emplir (3) selon la revendication 5, dans laquelle la valve d'alimentation en air (10) est configurée pour se déplacer entre
- une première position (10a) qui assure l'apport de l'air vers la buse venturi (9) tout en fermant la conduite de décharge de vide (13), et
- une deuxième position (10b) qui empêche l'air d'être apporté vers la buse venturi (9) tout en ouvrant la conduite de décharge de vide (13), ce qui assure une pression atmosphérique dans la conduite de décharge de vide (13).

7. Machine à emplir (3) selon l'une quelconque des revendications précédentes, comprenant :
un drain (21) configuré pour évacuer le fluide de stérilisation du système (1),
une valve de chargement (37) raccordée entre le réservoir de remplissage (4) et le réservoir de pulvérisation (5) pour assurer l'aspiration du fluide de stérilisation (2) jusque dans le réservoir de pulvérisation (5) lorsque la valve de chargement (37) est ouverte, et
une conduite de drainage de fluide (41) raccordée entre le réservoir de remplissage (4) et le drain (21) pour évacuer le fluide de stérilisation (2) du réservoir de pulvérisation (5) lorsque la valve de chargement (37) est fermée.

8. Machine à emplir (3) selon la revendication 7, comprenant une valve de drainage (38) agencée le long de la conduite de drainage de fluide (41) pour vider le réservoir de remplissage (4) lorsque la valve de drainage (38) est ouverte et que la valve de chargement (37) est fermée et, la buse venturi (9) ayant une sortie d'air (19) raccordée au drain (21) par l'intermédiaire d'une conduite de sortie d'air (20) pour évacuer l'air de la buse venturi (9).

9. Machine à emplir (3) selon l'une quelconque des revendications précédentes, dans laquelle ladite machine à emplir (3) est configurée pour former des emballages et emplir les emballages du produit alimentaire liquide.

10. Machine à emplir (3) selon l'une quelconque des revendications précédentes, dans laquelle le réservoir de pulvérisation (5) inclut un transducteur de pression (35) configuré pour surveiller une pression à l'intérieur du réservoir de pulvérisation (5).

11. Machine à emplir (3) selon l'une quelconque des revendications précédentes, dans laquelle le réservoir de pulvérisation (5) inclut un relais dispositif de relais de niveau élevé (33) et un relais de niveau bas (34) qui sont chacun configurés pour détecter un niveau du fluide de stérilisation (2) dans le réservoir de pulvérisation (5).

12. Machine à emplir (3) selon l'une quelconque des revendications précédentes, comprenant :
une valve de régulation de pression (23) pour le réservoir de pulvérisation (5), configurée pour se déplacer entre
- une première position (23a) qui fournit un raccordement entre le réservoir de pulvérisation (5) et le générateur de vide (7), pour permettre la génération du vide dans le réservoir de pulvérisation (5), et
- une deuxième position (23b) qui fournit un raccordement entre le réservoir de pulvérisation (5) et une valve d'alimentation en air (25), pour permettre à l'air sous pression d'entrer dans le réservoir de pulvérisation (5), destiné à être utilisé pour transporter un fluide de stérilisation (2), et
une valve de régulation de fluide de stérilisation (30) qui est configurée pour se déplacer entre
- une première position (30a) qui fournit un raccordement entre le réservoir de pulvérisation (5) et le réservoir de remplissage (4), pour permettre au fluide de stérilisation (2) d'être aspiré du réservoir de remplissage (4) et jusque dans le réservoir de pulvérisation (5), et
- une deuxième position (30b) qui fournit un raccordement entre le réservoir de pulvérisation (5) et la machine à emplir (3), pour permettre au fluide de stérilisation (2) d'être transporté vers la machine à emplir (3).

13. Machine à emplir (3) selon l'une quelconque des revendications précédentes, dans laquelle le système (1) peut fonctionner dans un mode emplissage et dans un mode pulvérisation qui est distinct du mode emplissage, dans laquelle :
dans le mode emplissage, le générateur de vide (7) est en communication fluidique avec le réservoir de pulvérisation (5) pour générer le vide et aspirer le fluide de stérilisation (2) jusque dans le réservoir de pulvérisation (5) pour emplir le réservoir de pulvérisation (5), et une communication fluidique entre la machine à emplir (3) et le réservoir de pulvérisation (5) est fermée ;
dans le mode pulvérisation, la communication fluidique entre le générateur de vide (7) et le réservoir de pulvérisation (5) est fermée et le réservoir de pulvérisation (5) est mis sous pression, de telle que la pression dans le réservoir de pulvérisation (5) pousse le fluide de stérilisation (2) qui a été aspiré jusque dans le réservoir de pulvérisation (5) pendant le mode emplissage à être évacué du réservoir de pulvérisation (5) et transporté vers la buse de pulvérisation pour la machine à emplir (3).

14. Procédé (45) de manipulation d'un fluide de stérilisation (2) destiné à être utilisé pour une machine à emplir (3) qui est agencée pour emplir des emballages d'un produit alimentaire liquide, le procédé (45) comprenant :
l'aspiration (46) du fluide de stérilisation (2) d'un réservoir de remplissage (4),
la réception (47) du fluide de stérilisation (2) dans un réservoir de pulvérisation (5) raccordé de façon fluidique au réservoir de remplissage (4), et
le transport (48) du fluide de stérilisation (2) vers la machine à emplir (3),
le fluide de stérilisation (2) étant transporté vers une buse de pulvérisation de la machine à emplir (3) qui est agencée pour pulvériser diverses parties de la machine à emplir (3),
l'aspiration (46) comprenant la génération d'un vide dans le réservoir de pulvérisation (5) par l'intermédiaire d'un générateur de vide (7) raccordé de façon fluidique au réservoir de pulvérisation (5).

15. Procédé (45) selon la revendication 14, dans lequel le fluide de stérilisation (2) est du peroxyde d'hydrogène.
